(19) Europäisches Patentamt
European Patent Office
Office européen des brevets

(11) Veröffentlichungsnummer: **0 143 403 B1**

# (12) EUROPÄISCHE PATENTSCHRIFT

(45) Veröffentlichungstag der Patentschrift: **10.04.91**

(51) Int. Cl.[5]: **C12P 13/00**, C12P 11/00, C07C 303/36, A61K 31/16

(21) Anmeldenummer: **84113813.4**

(22) Anmeldetag: **15.11.84**

## (54) Verfahren zur Herstellung von Indanyl-Derivaten und deren Verwendung.

(30) Priorität: **28.11.83 DE 3343331**

(43) Veröffentlichungstag der Anmeldung:
**05.06.85 Patentblatt 85/23**

(45) Bekanntmachung des Hinweises auf die Patenterteilung:
**10.04.91 Patentblatt 91/15**

(84) Benannte Vertragsstaaten:
**AT BE CH DE FR GB IT LI LU NL SE**

(56) Entgegenhaltungen:
**EP-A- 0 009 554**
**EP-A- 0 056 956**
**EP-A- 0 088 282**

**CHEMICAL ABSTRACTS, Band 99, Nr. 25, 19. Dezember 1983, Seite 11, Zusammenfassung Nr. 205520k, Columbus, Ohio, US; W. KRAUSE et al.: "Pharmacokinetics and biotransformation of methanesulfonanilides with antiinflammatory activity in the rat and monkey. Comparison with piroxicam", & XENOBIOTICA 1983, 13(5), 265-272**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Petzoldt, Karl, Dr.**
**Flachsweg 10**
**W-1000 Berlin 38(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft das in den Patentansprüchen gekennzeichnete Verfahren und die Verwendung der dabei erhaltenen Verfahrensprodukte.

Das erfindungsgemäße Verfahren wird unter den Bedingungen durchgeführt, die man üblicherweise bei der mikrobiologischen Hydroxylierung von Substraten mit Pilzkulturen anwendet. So werden zunächst in allgemein üblichen Vorversuchen die günstigsten Fermentationsbedingungen, wie zum Beispiel Auswahl des günstigsten Nährmediums, des geeigneten Substratlösungs-oder Suspensionsmittels, der Substratkonzentration, der technischen Bedingungen wie Temperatur, Belüftung, pH-Wert und der optimalen Zeiten für Germination, Substratzugabe und Substratkontakt am Enzym des Mikroorganismus analytisch, insbesondere dünnschichtchromatographisch, ermittelt.

Dabei hat sich gezeigt, daß es zweckmäßig ist, Konzentrationen von etwa 100 bis 2000 mg Substrat pro Liter Nährmedium einzusetzen. Der pH-Wert wird vorzugsweise auf einen Wert im Bereich von 5 bis 7 eingestellt. Die Züchtungstemperatur liegt im Bereich von 20 - 40° C, vorzugsweise von 25 - 35° C. Zur Belüftung werden vorzugsweise 0.5 bis 5 Liter Luft pro Minute pro Liter Kulturbrühe zugeführt. Die Umwandlung des Substrats wird zweckmäßigerweise durch Analysen von Probeextrakten verfolgt.

Die für diese Fermentation benötigten Mikrorganismen stehen der Fachwelt bei anerkannten Mikroorganismen-Sammlungen zur freien Verfügung. Geeignete Stämme sind beispielsweise Aspergillus niger ATCC 10577 und NRRL 3228 oder Polyporus tulipiferus CBS 43148.

Nach erfolgter Fermentation werden die Fermentationsprodukte in an sich bekannter Weise isoliert. Die Isolierung kann zum Beispiel in der Weise erfolgen, daß man die Fermentationsansätze mit einem nicht mit Wasser mischbaren organischen Lösungsmittel wie Äthylacetat, Butylacetat oder Methylisobutylketon, extrahiert, die Extrakte einengt und die so erhaltenen Rohprodukte gegebenenfalls durch Chromatographie und/oder Kristallisation reinigt.

Die erfindungsgemäßen Verbindungen zeichnen sich durch eine gute antiphlogistische Wirksamkeit aus.

Darüberhinaus zeichnen sich die erfindungsgemäßen Substanzen durch eine analgetische, antidysmenorrhöische, antipyretische thrombocytenaggregationshemmende und diuretische Wirksamkeit aus. Darüberhinaus ist bemerkenswert, daß diese Substanzen die Prostaglandinsynthese kaum hemmen. Ein besonderer Vorzug dieser Verbindungen ist die große Dissoziation zwischen therapeutischer Wirksamkeit und unerwünschter Nebenwirkung (insbesondere Ulcerogenese).

Somit eignen sich die neuen Verbindungen in Kombination mit den in der galenischen Pharmazie üblichen Trägermitteln zur Behandlung von Erkrankungen des rheumatischen Formenkreises (wie der rheumatischen Arthritis, Ostearthritis oder ankylosierenden Spondalitis) Asthma bronchiale, Heufieber u.a..

Bemerkenswert ist ferner, daß sich die Indanyl-Derivate der allgemeinen Formel I gemäß Anspruch 1 darüberhinaus auch zur Behandlung von Migräne und von Dysmenorhoe eignen und das Thromboserisiko mindern.

Überraschenderweise gibt es unter den erfindungsgemäßen Indanyl-Derivaten auch solche, die zusätzlich noch eine ausgeprägte antiulcerogene sowie tumorhemmende Wirksamkeit besitzen.

Die Herstellung der Arzneimittelspezialitäten erfolgt in üblicher Weise, indem man die Wirkstoffe mit geeigneten Zusätzen, Trägersubstanzen und Geschmackskorrigentien in die gewünschten Applikationsformen wie Tabletten, Dragees, Kapseln, Lösungen, Inhalationsmitteln usw. überführt.

Für die orale Anwendung eignen sich insbesondere Tabletten, Dragees und Kapseln, welche beispielsweise 1 bis 250 mg Wirkstoff und 50 mg bis 2 g pharmakologisch unwirksamen Trägers, wie zum Beispiel Laktose, Amylose, Talkum, Gelatine, Magnesiumstearat und ähnliches, sowie die üblichen Zusätze enthalten.

Die Ausgangsverbindungen für das erfindungsgemäße Verfahren gemäß Anspruch 1 sind bekannt oder können in an sich bekannter Weise hergestellt werden.

Die nachfolgenden Ausführungsbeispiele dienen zur Erläuterung des erfindungsgemäßen Verfahrens.

### Beispiel 1

Ein 2 Liter Erlenmeyerkolben, der 500 ml einer sterilen wässrigen Nährlösung enthaltend

3,0 % Glucose
1,0 % Corn steep liquor
0,2 % Natriumnitrat
0,1 % Kaliumdihydrogenphosphat
0,2 % Dikaliumhydrogenphosphat

0,05 % Magnesiumsulfat-heptahydrat
0,002 % Eisen(II)-sulfat-heptahydrat
0,05 % Kaliumchlorid
wird mit einer Schrägagar-Kultur des Stammes Aspergillus niger ATCC 10577 oder Aspergillus niger NRRL 3228 beimpft und 60 Stunden lang bei 28° C geschüttelt.

Dann wird diese Vorkultur unter sterilen Bedingungen in einen 20 Liter Vorfermenter überführt, der mit 15 Liter der gleichen Nährlösung beschickt ist. Unter Zugabe von Silicon SH als Antischaummittel wird bei 29° C und 0,7 bar Überdruck unter Belüftung (15 Liter pro Minute) und Rühren (220 Umdrehungen pro Minute) 24 Stunden lang germiniert.

Danach werden 0,9 Liter dieser Kultur unter sterilen Bedingungen entnommen und damit ein 20 Liter Hauptfermenter beimpft, der 14 Liter des obengenannten Nährmediums enthält. Nach einer Anwachsphase von 12 Stunden unter Vorfermenterbedingungen wird eine sterile Lösung von 3,0 g N-[1-Oxo-6-phenoxyindan-5-yl]-methansulfonamid in 60 ml Dimethylformamid hinzugegeben und unter den oben angegebenen Bedingungen 48 Stunden lang fermentiert.

Dann extrahiert man die Kulturbrühe zweimal mit je 10 Liter Methylisobutylketon, engt den Extrakt im Vakuum ein, chromatographiert den Rückstand über eine Kieselgelsäule mittels Dichlormethan-Aceton-Gradienten und erhält nach Umkristallisation aus Aceton-Diisopropylether 1,74 g N-[1-Oxo-3β-hydroxy-6-phenoxyindan-5-yl]-methansulfonamid vom Schmelzpunkt 148-150° C.

Beispiel 2

Unter den Bedingungen des Beispiels 1 werden bei Verwendung des Mikroorganismus Polyporus tulipiferus CBC 43148 3,0 g N-[-1-Oxo-6-(2.4-difluorphenoxy)-indan-5-yl]-methansulfonamid in 46 Stunden Kontaktzeit hydroxyliert. Nach Aufarbeitung und säulenchromatographischer Reinigung des Rohprodukts an Kieselgel erhält man 0,96 g N-[1-Oxo-3ß-hydroxy-6-(2,4-difluorphenoxy)-indan-5-yl]-methansulfonamid in Form eines farblosen Öls, welches nach mehrtägigem Stehen im Kühlschrank durchkristallisiert. Schmelzpunkt 83 - 86° C.

## Ansprüche

**1.** Verfahren zur Herstellung von Indanyl-Derivaten der allgemeinen Formel I

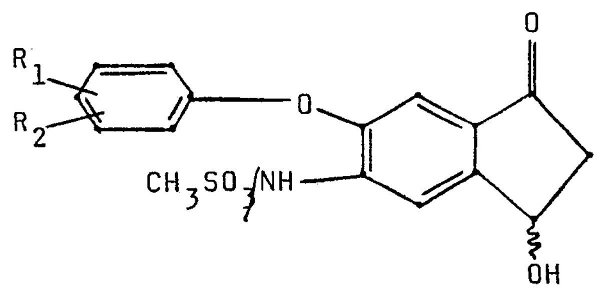

(I),

worin

$R_1$ und $R_2$ gleich oder verschieden sind und jeweils ein Wasserstoffatom, ein Fluoratom oder ein Chloratom bedeuten,
dadurch gekennzeichnet, daß man ein Indanyl-Derivat der allgemeinen Formel II

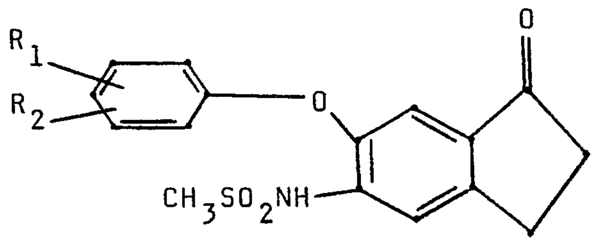

(II),

worin

$R_1$ und $R_2$ die im Anspruch 1 genannte Bedeutung besitzen mit einer lebenden Kultur von Aspergillus niger oder Polyporus tulipiferus fermentiert.

2. N-[1-Oxo-3β-hydroxy-6-phenoxyindan-5-yl]-methansulfonamid zur Behandlung entzündlicher oder allergischer Erkrankungen des Menschen.

3. N-[1-Oxo-3β-hydroxy-6-(2,4-difluorphenoxy)-indan-5-yl]-methansulfonamid zur Behandlung entzündlicher oder allergischer Erkrankungen des Menschen.

## Claims

1. Process for the preparation of indanyl derivatives of the general formula I wherein

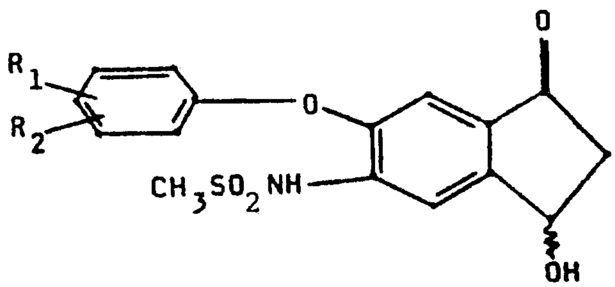

(I)

$R_1$ and $R_2$ are identical or different and each represents a hydrogen atom, a fluorine atom or a chlorine atom,

characterised in that an indanyl derivative of the general formula II wherein

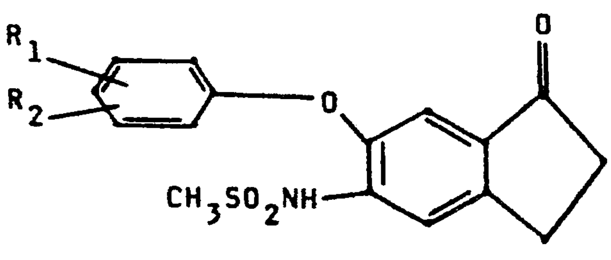

(II),

$R_1$ and $R_2$ have the meanings given in claim 1,

is fermented with a living culture of Aspergillus niger or Polyporus tulipiferus .

2. N-[1-oxo-3β-hydroxy-6-phenoxyindan-5-yl]-methanesulphonamide for the treatment of inflammatory or allergic disorders in humans.

3. N-[1-oxo-3β-hydroxy-6-(2,4-difluorophenoxy)-indan-5-yl]-methanesulphonamide for the treatment of inflammatory or allergic disorders in humans.

## Revendications

1. Procédé pour préparer des composés indanyliques répondant à la formule générale I :

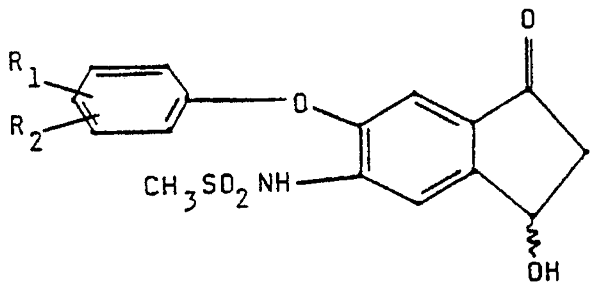

(I),

dans laquelle $R_1$ et $R_2$ représentent chacun, indépendamment l'un de l'autre, un atome d'hydrogène, de fluor ou de chlore, procédé caractérisé en ce qu'on fait fermenter un composé indanylique répondant à la formule générale II :

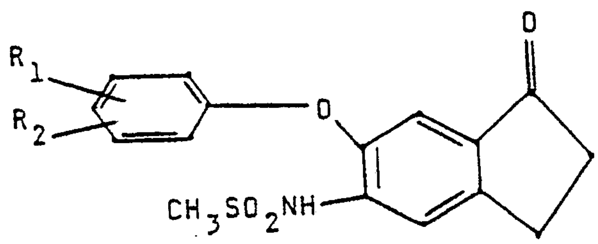

(II),

dans laquelle $R_1$ et $R_2$ ont les significations qui leur ont été données ci-dessus, avec une culture vivante d'Aspergillus niger ou de Polyporus tulipiferus.

2. N-[Oxo-1 hydroxy-3β phénoxy-6 indanyl-5]-méthane-sulfonamide pour le traitement de maladies inflammatoires ou allergiques de l'homme.

3. N-[Oxo-1 hydroxy-3β (difluoro-2,4 phénoxy)-6 indanyl 5]-méthane-sulfonamide pour le traitement de maladies inflammatoires ou allergiques de l'homme.